# EUROPEAN PATENT SPECIFICATION

(11) **EP 3 593 852 B1**
(45) Date of publication and mention of the grant of the patent: **15.09.2021**
(21) Application number: 19191331.8
(22) Date of filing: 26.02.2016
(51) Int. Cl.: A61M 25/06, A61M 39/22, A61M 25/00, A61M 39/26

(54) **IV CATHETER ASSEMBLIES WITH INJECTION PORTS**
IV-KATHETERANORDNUNGEN MIT INJEKTIONSÖFFNUNGEN
ENSEMBLES DE CATHÉTER IV AVEC DES ORIFICES D'INJECTION

(30) Priority: 26.02.2015 GB 201503230; 26.02.2015 GB 201503249
(43) Date of publication of application: 15.01.2020
(62) Divisional of application: 16706378.3
(73) Proprietor: B. Braun Melsungen AG, 34212 Melsungen (DE)
(72) Inventor: Lim, Siew Ping, 11600 Georgetown (Penang) (MY); Bin Tajudin, Riduan, 11900 Bayan Lepas (Penang) (MY); Tan, Soo Yong, 11900 Bayan Lepas (Penang) (MY); Phang, Chee Mun, 11900 Bayan Lepas (Penang) (MY); Woehr, Kevin, 34587 Felsberg (DE)
(74) Representative: Kinkeldey, Daniela

(56) References cited:
- EP-A1- 0 015 443
- WO-A1-96/40359
- WO-A1-2016/007440
- DE-U1- 7 812 248
- US-A- 5 098 410
- US-A- 5 618 268
- US-A1- 2004 181 192
- US-A1- 2012 035 552

## Description

The present disclosure generally relates to intravenous catheters and more particularly to intravenous catheters with injection ports having aspiration and/or injection capabilities.

After intravenous (IV) catheterization, it may be necessary to deliver a solution such as an anaesthetic through the catheter hub to the patient. One point of delivery is at an injection port integrated with the catheter hub of the IV catheter. The catheter hub is typically secured to the patient by taping the catheter hub to the patient's skin using IV dressing and/or medical tape. Typically, IV solution flows into a proximal end of the catheter hub and out a distal end of the catheter hub through a catheter tube and into the patient's vein. The injection port extends from the catheter hub and allows delivery of medicament into the solution stream without interrupting the flow of IV solution or requiring removal or assembly of parts which could cause discomfort to the patient.

A port cap assembly is often fitted onto the injection port to maintain cleanliness and protects against infection.

EP 0 015 443 discloses a medical apparatus for the introduction or removal of liquid from a patient by means of a syringe.

EP 0 947 212 discloses a self-priming, needle free 'Y'-adapter for use with intravenous delivery systems.

A valve assembly for medical fluid circuits is disclosed in EP 0 472 088.

A laparoscopic cannula is described in US 4,112,932.

An adapter cap attachment for an infusion catheter is shown and described in US 5,098,410.

More recently, US 2004/0181182 discloses a vascular access device and a method for using the same.

A cap having a triangular form for an intravenous catheter is shown and described in EP 1 658 875.

US2012/0035552 A1 discloses a safety intravenous catheter.

One aspect of the present disclosure includes a ported catheter having a low profile. Another aspect of the present disclosure includes a ported catheter having an injection port that is offset from a lengthwise axis of the catheter. A still further aspect of the present disclosure is a ported catheter that is sized and shaped for opening a collapsible valve through physical abutment with the valve so that aspiration through the injection port is possible. Yet another aspect of the present disclosure is a quick and simple removal and replacement of a cap assembly from the injection port to minimize discomfort to the patient caused by opening and closing the cap, which could cause the puncture site to move if opened in a disadvantaged orientation. Further, when removing the port cap assembly prior to medicament delivery, the port cap assembly should be secured to the catheter hub to prevent being misplaced or contaminating contact.

In a first aspect, the present disclosure provides a ported catheter assembly, comprising:
a catheter hub having a distal end, an interior cavity, and a proximal end;
a port extending from the catheter hub and communicating with the interior cavity of the catheter hub; and
a catheter extending from the distal end of the catheter hub;
wherein the port is radially offset from the catheter hub, such that a central axis of the port and a central axis of the interior cavity of the catheter hub do not intersect and are offset from each other.

The catheter assembly of the present disclosure comprises a port. The port has a bore or lumen therethrough, which is provided with a female Luer taper in its outer portion, as is known in the art. In use, a fluid, such as a medicament, may be injected into the interior cavity of the catheter hub by insertion of a suitable instrument, such as a syringe or a connector of an IV drip line with a male Luer taper, into the port.

In the catheter assembly of this aspect, the port has a central axis that is offset from the central longitudinal axis of the interior cavity of the catheter hub, such that the two axes do not intersect. The port may have its central axis extending at any angle to the central axis of the cavity of the catheter hub. In one preferred alternative, the central axis of the port extends substantially parallel to a plane extending perpendicular to the central axis of the interior cavity of the catheter hub.

The port may open into and be in communication with the interior cavity of the catheter hub. In this way, fluid may pass freely between the port and the interior cavity of the catheter hub in either direction. Alternatively, a seal may be formed between the port and the interior cavity of the catheter hub. More preferably, a valve is provided to provide a seal between the port and the interior cavity of the catheter hub. Preferably, the valve is disposed in the interior cavity of the catheter hub. In this way, the flow of fluid between the port and the interior cavity of the catheter hub may be controlled and limited or prevented. In particular, the valve may be a one-way valve and operate to allow fluid to enter the interior cavity of the catheter hub from the port, but to prevent fluid flowing in the reverse direction from the interior cavity of the catheter hub into the port. In this way, a fluid to be injected may be applied to the port, for example by a syringe or the like, and caused to flow into the catheter hub and the catheter. However, fluid, such as blood, is prevented from leaving the interior cavity of the catheter hub through the port by the action of the valve closing.

Any suitable arrangement for the valve may be employed. In one preferred arrangement, the valve is arranged to move inwardly under pressure applied to the port and open, thereby allowing fluid to pass from the port to the interior of the cavity. The valve may move inwardly under the action of fluid pressure within the port. Alternatively, or in addition, the valve may be arranged to move inwardly under the action of an instrument, such as a syringe, pressing against the valve when inserted into the port.

A valve opener may be provided in the port. The valve opener is moveable within the port, such that an instrument, such as a syringe, connected to the port urges the valve opener into contact with the valve, thereby opening the valve to allow the passage of fluid from the port into the interior cavity of the catheter hub. The valve is preferably resilient, the resilience of the valve material urging the valve into the closed position.

In another alternative, the valve may be arranged to prevent fluid from either entering or exiting the interior cavity of the catheter hub through the port.

Suitable materials for forming the valve are known in the art, in particular resilient materials. One preferred material is silicone.

The port may be provided with a cap assembly. A preferred cap assembly comprises a ring and a sealing top. The sealing top is preferably connected to the ring, more preferably by a hinge, for example a living hinge. The ring may be rotatably fixed to the port, with the cap assembly being rotatable between an open position, in which the port is open, and a closed position, in which the port is closed.

In one preferred arrangement, the port is provided with a track defined circumferentially around an exterior portion of the port. The ring is provided with a tab extending from an inner surface thereof to engage with the track. Movement of the ring around the port is limited by the length of the track. In this way, rotational movement of the ring is limited by the tab engaging with the ends of the track.

The port may be provided with a notch, the sealing top being provided with a chamfered protruding ring to engage with the notch. When the protruding ring is engaged with the notch, the port is covered and the cap assembly is in the closed position. When the chamfered ring is not engaged with the notch, the cap assembly is in the open position.

In one preferred alternative, the catheter hub is provide with a pair of wings extending therefrom. In particular, the wings preferably extend laterally from opposing sides of the catheter hub. The wings may be rigid. Alternatively, each wing may be provided with a hinge, such as a living hinge, whereby each wing may be folded with respect to the catheter hub. The wings may be used to secure the catheter hub to the patient, for example using adhesive tape applied over the wings and to the skin of the patient.

In addition, the present disclosure provides a ported catheter assembly, comprising:
a catheter hub comprising a catheter body having a distal end, an interior cavity, and a proximal end, and an injection port extending from the catheter body and having a lumen communicating with the interior cavity of the catheter body;
a catheter fixed to a distal end of the catheter body; and
a valve disposed in the interior cavity of the catheter body forming a deformable seal between the injection port and the interior cavity of the catheter body;
wherein the injection port is offset from the catheter body, such that a central axis of the port and a central axis of the interior cavity of the catheter hub do not intersect and are offset from each other.

Details of the assembly and preferred arrangements are as described above.

The present disclosure also provides a method of using a ported catheter assembly, the method comprising:
attaching a catheter to a patient, the catheter connected to a catheter hub having an interior cavity and a port communicating with the interior cavity of the catheter hub, the port being radially offset from the catheter hub, such that a central axis of the port and a central axis of the interior cavity of the catheter hub do not intersect and are offset from each other;
breaking a seal formed between the port and the interior cavity of the catheter hub;
injecting a solution to the patient via the port; and
restoring the seal after solution has been injected.

Details and preferred embodiments for the ported catheter assembly being used are as described hereinbefore.

According to the present invention, an improved arrangement of a ported catheter assembly is now provided. The improved assembly provides significant advantages in the delivery or removal of a fluid to the catheter through the catheter hub by means of the port. In particular, the improved catheter assembly allows for fluid to be delivered to the catheter through the port and for fluid to be withdrawn from the catheter through the port, thereby increasing the options available for a medical practitioner when using the assembly to deliver or remove fluid from a patient.

Accordingly, the present invention provides a ported catheter assembly comprising the features of claim 1.

The catheter assembly of the present invention has a port in communication with the interior cavity of the catheter hub. The port has a bore or lumen extending therethrough, which is provided with a female Luer taper in its outer end portion for connection to an instrument having a corresponding male Luer taper, as is known in the art. The communication of the lumen of the port with the interior cavity of the catheter hub is sealed by means of a valve. In use, a fluid, such as a medicament, may be injected into the interior cavity of the catheter hub by insertion of a suitable instrument, such as a syringe or a connector of an IV drip line with a male Luer taper, into the port. In addition, the interior cavity of the catheter hub may be aspirated through the port, for example to withdraw fluid, such as blood, from the catheter hub.

The port may have its central axis aligned with the central longitudinal axis of the interior cavity of the catheter hub, as is known in the art. Alternatively, the port has a central axis that is offset from the central longitudinal axis of the interior cavity of the catheter hub, such that the two axes do not intersect. This arrangement allows the overall height of the assembly to be reduced, compared with known catheter assemblies. The port may have its central axis extending at any angle to the central axis of the cavity of the catheter hub. In one preferred embodiment, the central axis of the port extends substantially parallel to a plane extending perpendicular to the central axis of the interior cavity of the catheter hub.

The port opens into and is in communication with the interior cavity of the catheter hub. In this way, fluid may pass freely between the port and the interior cavity of the catheter hub in either direction. A valve provides a seal between the port and the interior cavity of the catheter hub. Preferably, the valve is disposed in the interior cavity of the catheter hub.

The valve functions to allow the flow of fluid between the port and the interior cavity of the catheter hub to be controlled and limited or prevented. In particular, the valve is a two-way valve and operates to allow fluid to enter the interior cavity of the catheter hub from the port and also to allow fluid to flow in the reverse direction, that is from the interior cavity of the catheter hub into the port. In this way, a fluid to be injected may be applied to the port, for example by a syringe or the like, and caused to flow into the catheter hub and the catheter. In addition, fluid, such as blood, may be withdrawn from the interior cavity of the catheter hub through the port by the action of the valve closing.

Any suitable arrangement for the valve may be employed. In one preferred arrangement, the valve is arranged to have a portion that moves inwardly and open, thereby allowing fluid to pass between the port and the interior cavity of the catheter hub.

According to an alternative not forming part of the invention, the valve may move from the closed position to the open position under the action of fluid pressure within the port. For example, an instrument, such as a syringe, may be inserted into the port and used to provide a fluid under pressure to the port, the fluid pressure causing the valve to move inwardly and open.

According to the invention, the valve may be arranged to move from the closed position to the open position under the action of an instrument, such as a syringe, pressing against the valve when inserted into the port.

Alternatively, according to the invention, a valve opener is provided in the lumen of the port. The valve opener is moveable within the lumen of the port, such that an instrument, for example a syringe, connected to the port urges the valve opener into contact with the valve, thereby opening the valve to allow the passage of fluid between the port and the interior cavity of the catheter hub.

The valve opener may comprise any suitable shape, which may be contacted by an instrument inserted into the port and contact the valve to move the valve from the closed position to the open position. Fluid flowing through the port between the instrument and the interior cavity of the catheter hub may flow through and/or around the valve opener. In one embodiment, the valve opener comprises a valve opener body having a bore therethrough for the passage of fluid between the instrument and the interior cavity of the catheter hub. The valve opener body is preferably generally cylindrical, with the bore therethrough preferably being cylindrical.

In one embodiment, the valve opener comprises a valve opener body, preferably a generally cylindrical body, having a bore therethrough, again preferably a cylindrical bore, with the valve opener body having an actuating end that contacts the valve. The actuating end preferably comprises one or more openings extending therefrom along a portion of the valve body, preferably arched openings. In this way, the actuating end of the valve opener is prevented from sealing against the valve and allows fluid to flow freely through the openings. The actuating end portion of the valve opener is preferably tapered. In one embodiment, the opening of the port into the interior cavity of the catheter hub has a diameter smaller than the diameter of the lumen of the port, such that a shoulder is defined within the lumen. The diameter of the valve body is greater than the diameter of the opening. The taper on the actuating end portion of the valve opener body is sized to allow the actuating end to move through the opening and contact the valve. In this way, movement of the valve opener towards the valve is limited by the valve body contacting the shoulder, thereby limiting the range of movement of the valve opener.

In one preferred embodiment, the valve comprises a generally cylindrical valve body disposed in the interior cavity of the catheter hub. The valve body has a longitudinal axis that is aligned with the longitudinal axis of the interior cavity of the catheter hub. The outer surface of the valve body is preferably in contact with the inner surface of the cavity in the catheter hub and extends across the inner opening of the port. In this way, the valve body provides a fluid seal with the inner surface of the interior cavity of the catheter hub and seals the inner opening of the port.

The valve is preferably biased into the closed position, that is the position in which fluid is prevented from flowing between the interior cavity of the catheter hub and the port. The valve is preferably resilient, the resilience of the valve material urging the valve into the closed position.

Suitable materials for forming the valve are known in the art, in particular resilient materials. One preferred material is silicone.

The port may be provided with a cap assembly. A preferred cap assembly comprises a ring and a sealing top. The sealing top is preferably connected to the ring, more preferably by a hinge, for example a living hinge. The ring may be rotatably fixed to the port, with the cap assembly being rotatable between an open position, in which the port is open, and a closed position, in which the port is closed.

In one preferred arrangement, the port is provided with a track defined circumferentially around an exterior portion of the port. The ring is provided with a tab extending from an inner surface thereof to engage with the track. Movement of the ring around the port is limited by the length of the track. In this way, rotational movement of the ring is limited by the tab engaging with the ends of the track.

The port may be provided with a notch, the sealing top being provided with a chamfered protruding ring to engage with the notch. When the protruding ring is engaged with the notch, the port is covered and the cap assembly is in the closed position. When the chamfered ring is not engaged with the notch, the cap assembly is in the open position.

In one preferred embodiment, the catheter hub is provide with a pair of wings extending therefrom. In particular, the wings preferably extend laterally from opposing sides of the catheter hub. The wings may be rigid. Alternatively, the wings may each be provided with a hinge, such as a living hinge, to allow each wing to be folded with respect to the catheter hub. The wings may be used to secure the catheter hub to the patient, for example using adhesive tape applied over the wings and to the skin of the patient.

In addition, the present disclosure provides a method of using a ported catheter assembly, the method comprising:
attaching a catheter of the ported catheter assembly to a patient, the catheter connected to a catheter hub of the ported catheter assembly having an interior cavity and a port communicating with the interior cavity of the catheter hub;
moving a valve of the ported catheter assembly from a closed position to an open position, thereby breaking a seal formed between the port and the interior cavity of the catheter hub;
transferring a fluid from the interior cavity of the catheter hub to the port; and restoring the valve to the closed position after fluid transfer has been completed.

The method may be used to inject fluid into the patient by means of a suitable instrument, such as a syringe, inserted into the port. The method may further comprise withdrawing fluid from the patient, again by a suitable instrument inserted into the port. When not in use, the valve closes and seals the port and prevents fluid flowing in either direction between the port and the interior cavity of the catheter hub.

Details and preferred examples for the method and the ported catheter assembly being used are as described hereinbefore.

These and other features and advantages of the present devices, systems, and methods will become appreciated as the same become better understood with reference to the specification, claims and appended drawings wherein:
Figure 1 is an isometric view of one embodiment of a ported catheter assembly having an offset injection port, the ported catheter assembly including a catheter tube, a catheter hub, and a port cap assembly;
Figure 2 is an exploded isometric view of the ported catheter assembly of Figure 1;
Figure 3 is a top view of the ported catheter assembly of Figure 1 shown without the port cap assembly;
Figure 4 is a side view of the ported catheter assembly of Figure 1;
Figure 5A is a back view of the ported catheter hub of Figure 1 showing the offset of the port;
Figure 5B is a back view of an in-line ported catheter assembly of the prior art;
Figure 6 is a cross-sectional view of the ported catheter assembly of Figure 4 taken along the line Y-Y in Figure 4 with a male medical implement inserted in the port;
Figure 7 is a cross-sectional view of the ported catheter assembly of Figure 4 taken along the line Z-Z in Figure 4;
Figure 8 is a cross-sectional view of an embodiment of the ported catheter assembly of Figure 4 taken along the line Y-Y in Figure 4 with a male medical implement inserted in the port and employing a valve opener;
Figure 9 is a cross-sectional view of another embodiment of the ported catheter assembly taken along the line Y-Y in Figure 4 with a male medical implement inserted in the port;
Figure 10 is a cross-sectional view of the ported catheter assembly of Figure 9 taken along the line Z-Z in Figure 4;
Figure 11 is an exploded isometric view of the ported catheter assembly having a centered injection port;
Figure 12 is a vertical cross-sectional view of the ported catheter assembly of Figure 11 along the line Y-Y of Figure 11 and shown with a male medical implement and a valve opener;
Figure 12a shows a side view of the valve opener of Figure 12;
Figure 13a is a vertical cross-sectional view of one embodiment of a ported catheter assembly having the offset injection port and Figure 13b is a vertical cross-sectional view of a ported catheter assembly having a centered injection port, for comparison purposes;
Figure 14 is a cross-sectional view of one example of a syringe for use with the port of the assembly;
Figure 15 is an isometric view of a port cap assembly;
Figures 16A and B are isometric views of a ported catheter assembly shown with a partial cut-away view of the port cap assembly in closed positions;
Figure 17 is a cross-sectional view of the top of the ported catheter assembly of Figure 4 taken along lines X-X in Figure 4 detailing rotation of the port cap assembly;
Figure 18 is a vertical cross-sectional view of the ported catheter assembly of Figure 4 with a spring clip needle guard and a needle in a ready position within the catheter hub; and
Figure 19 is a view of the needle and spring clip of Figure 18 in a protected position and removed from the catheter hub.

The detailed description set forth below in connection with the appended drawings is intended as a description of the presently preferred examples of ported catheter assemblies provided in accordance with aspects of the present devices, systems, and methods and is not intended to represent the only forms in which the present devices, systems, and methods may be constructed or utilized. The description sets forth the features and the steps for constructing and using the examples of the present devices, systems, and methods in connection with the illustrated examples. As denoted elsewhere herein, like element numbers are intended to indicate like or similar elements or features.

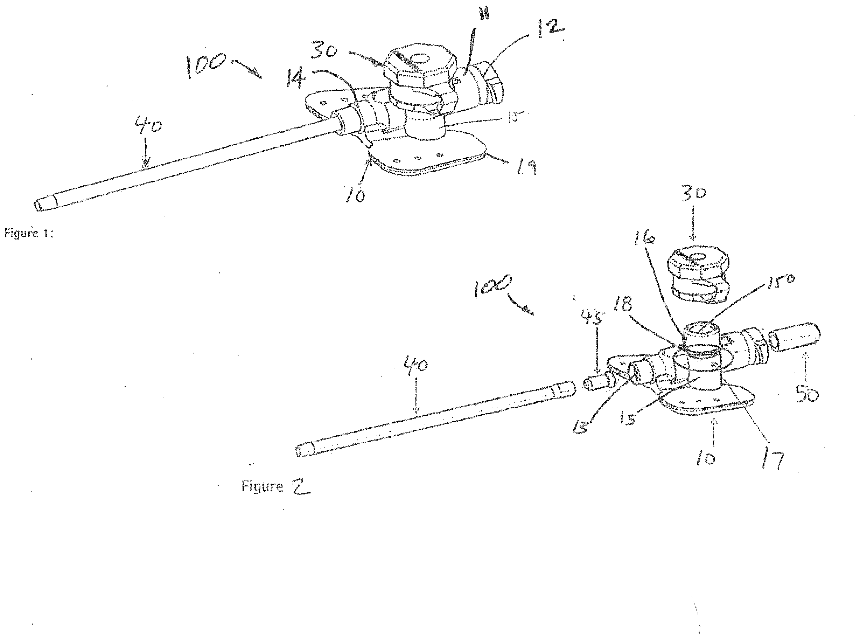

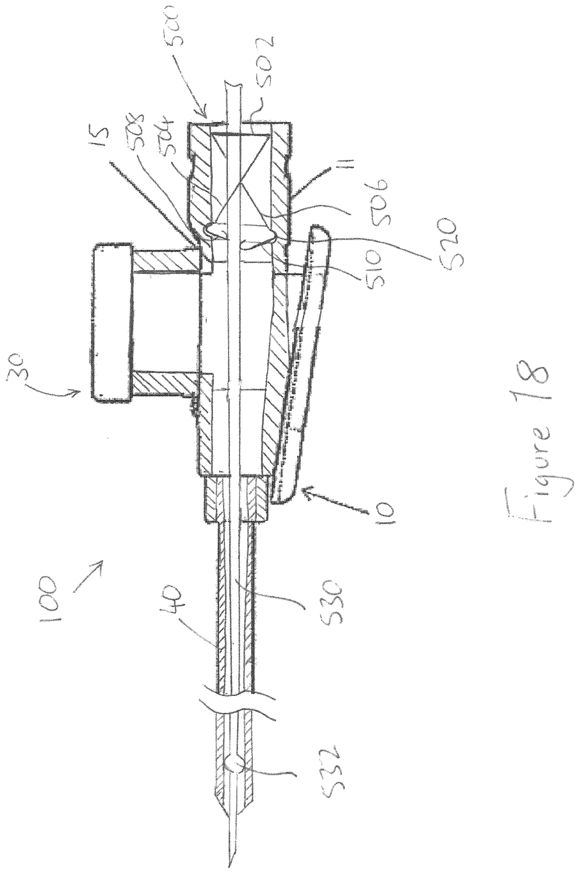

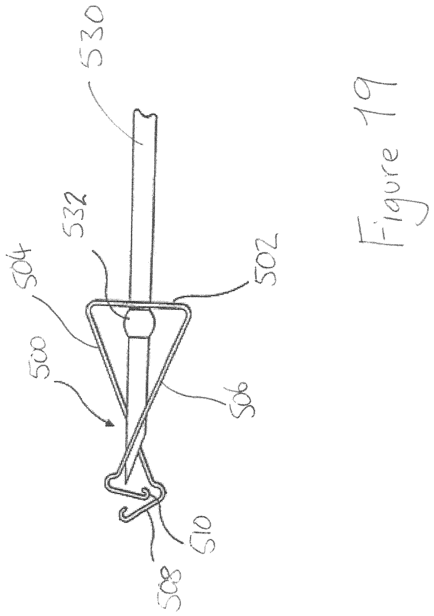

With reference now to Figures 1 and 2, an example of a ported catheter assembly 100 is shown including a catheter hub 10 having a catheter body 11 and an injection port 15 extending from the catheter body 11, a port cap assembly 30 capping the injection port 15, a valve 50 (see Figure 2) disposed in the interior of the catheter hub 10 to control the flow of fluids through the injection port 15, and a catheter tube 40 attached to and extending distally from the catheter hub 10. The ported catheter assembly 100 is shown without a needle hub and a needle for inserting the catheter tube 40 into a patient. Thus, the assembly shown in Figure 1 represents a used condition following successful venipuncture. Accordingly, an aspect of the present disclosure is understood to include a ported catheter hub with a catheter tube, a needle projecting through the catheter tube with the needle tip extending distally of the distal opening of the catheter tube, and a needle hub attached to the needle and located proximally of the catheter hub. In some examples, a third hub having a tip guard for covering the needle tip following successful venipuncture is provided between the catheter hub and the needle hub. In still other examples, the needle guard is provided with the needle hub so that following successful venipuncture, the needle and needle tip is retracted into the needle hub, such as by spring action. In still other examples, a spring clip is engaged inside the catheter hub until the needle tip is retracted inside the spring clip, which then collapses and blocks the needle tip while disengaging from the inside of the catheter hub. In this last example, the needle can have a change in contour proximal of the needle tip, which is blocked from passing through an opening in a proximal wall of the spring clip. An example of an assembly having a spring clip of this kind is shown in Figures 18 and 19 and discussed in more detail below.

The catheter hub 10 has a proximal end 12, a distal end 14 and an interior cavity 13 formed therebetween. The catheter tube 40 and the catheter hub 10 provide a fluid pathway to facilitate delivery of fluids or retrieval of fluids, such as for aspirating blood samples, from within the patient. A distal end of the catheter tube 40 is inserted into the patient and a proximal end of the catheter tube 40 is attached to a bushing 45. The bushing 45 is attached inside the distal end 14 of the interior cavity 13 of the catheter hub 10 to secure the catheter tube 40 to the catheter hub 10. The bushing 45 can also form a seal with the catheter hub 10 to prevent fluid from leaking out the distal end 14 of the catheter hub 10. For example, when the catheter 40 is inserted into the patient, solution such as medicament can flow from the proximal end 12 of the catheter hub 10 to the catheter tube 40 without leakage. The catheter hub 10 is configured for use with intravenous tubing to facilitate delivery of fluids, such as saline solution and/or medication, into or removal of fluids from the patient. As shown, the catheter hub 10 includes a catheter body 11 having an injection port 15 extending therefrom. As shown, the catheter hub 10 has a lengthwise axis and the injection port 15 has an axis that is located off-axis to the lengthwise axis. In another example, the axis of the injection port 15 is located on the axis of the catheter hub, such as being in-line, and preferably at a right angle therefrom, as further discussed below. A pair of wings 19 extends from the catheter body 11 for use to secure and stabilize the ported catheter assembly 100 to the patient following catheterization.

The catheter body 11 has an interior cavity or lumen 13 defined between its two ends, which can be in fluid communication with the injection port 15, as further discussed below. The proximal end 12 of the catheter body 11 has a female Luer connector having a female Luer taper and can have external threads. The female Luer connector is thus configured to matingly receive a male Luer connector, such as an IV line, a Luer access connector, a syringe tip, a vent plug, another known connector or future-developed IV devices. Each of these components can be sized and configured in conformity with at least some of the International Standards Organization (ISO) standards for female and male Luer connections under current or future standards. For discussion purposes, any one of these components or the class of these components may be referred to as a male medical implement.

The injection port 15 is configured for bolus injection, such as by receiving a syringe tip and medicament displaced by a syringe, and can be further configured for withdrawing fluid, such as blood, from the patient. The injection port 15 has a body defining a lumen 150, which can be in fluid communication with the interior cavity 13 of the catheter body 11. The lumen 150 of the injection port is incorporated with a Luer taper for receiving a male medical implement in a Luer fit arrangement. In other examples, threads are incorporated to the exterior of the injection port 15 for a Luer lock arrangement.

In the illustrated example, an outer surface of the injection port 15 incorporates a notch 16 configured to engage with a section of the port cap assembly 30 to create resistance to opening the port cap assembly 30 to access the injection port 15. In one example, the notch 16 presents a surface for interference fit with a projection or protrusion on the cap to form a locking mechanism for preventing the port cap assembly 30 from opening when in the closed position unless a separation force is applied to separate the lock. In the illustrated example, a track 17 is provided on the outer surface of the port. In one example, the track 17 is a channel engaging with an inner perimeter of the port cap assembly 30. In another example, the track 17 is a raised rib engaging a groove on the port cap assembly 30. The track 17 is a restricted path for the port cap assembly 30 and limits or restricts rotation of the port cap assembly 30 relative to the injection port 15 depending on the degree of rotation desired. For example, the track can be provided to limit the cap from rotating to within 90 degrees of an arc circle of the port, to within 180 degrees of an arc circle, or some higher or lower rotational range. In other examples, the cap is free to rotate along any arc length of the port and not delimited in any fashion. A flange 18 formed or provided on the injection port 15 of the catheter hub 10 can be configured for retaining the port cap assembly 30. The flange 18 can be chamfered to allow the port cap assembly 30 to snap into place onto the port 15 when assembling the port cap assembly to the catheter hub 10.

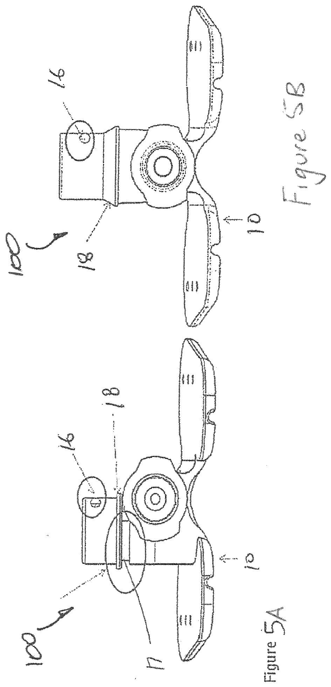

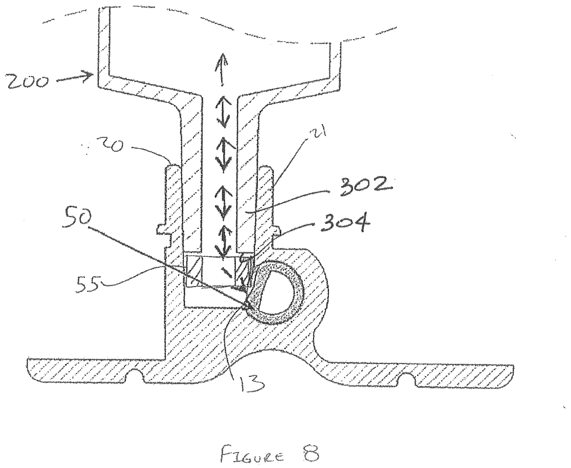

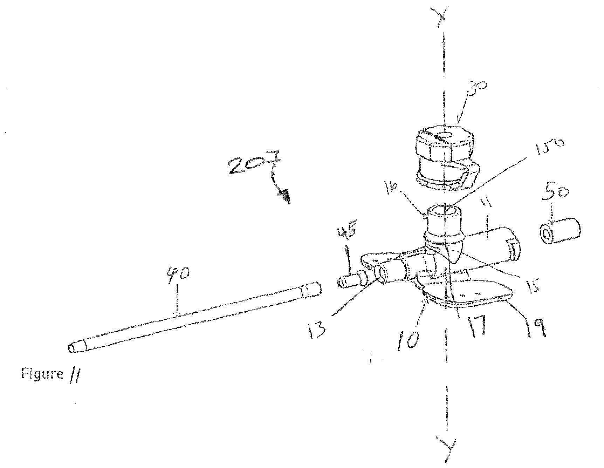

The injection port 15 is formed together with the catheter body 11, in particular a single component formed by techniques such as by plastic injection molding. In another example, the injection port 15 is attached to the catheter body 11 by welding, fusing, or other attachment means to integrate the two structures as a single integral unit. In other examples, the injection port is made from two or more port sections that are assembled together, such as by welding or snap-fit arrangement. Preferably the injection port 15 and the catheter body are made of the same biocompatible material. A valve opener 55 (see Figure 8) can be placed in the lumen 150 of the injection port 15 prior to assembling the port sections together. In other examples, the valve opener can be elastically deformed and forced into the port without resorting to a multi-port section configuration. The catheter assembly 10 can have an offset injection port or a centered injection port, such as axially in-line, as previously discussed and as further discussed below. For example, Figures 1 to 9 show a ported catheter assembly wherein the central axis of the injection port 15 is offset from the central axis of the catheter body 11 between the proximal end 12 and the distal end 14 of the catheter body 11. In another example as shown in Figures 11 to 12, the central axis of the injection port 15 directly intersects the axis of the catheter body 11 between the proximal end 12 and the distal end 14 of the catheter body 11.

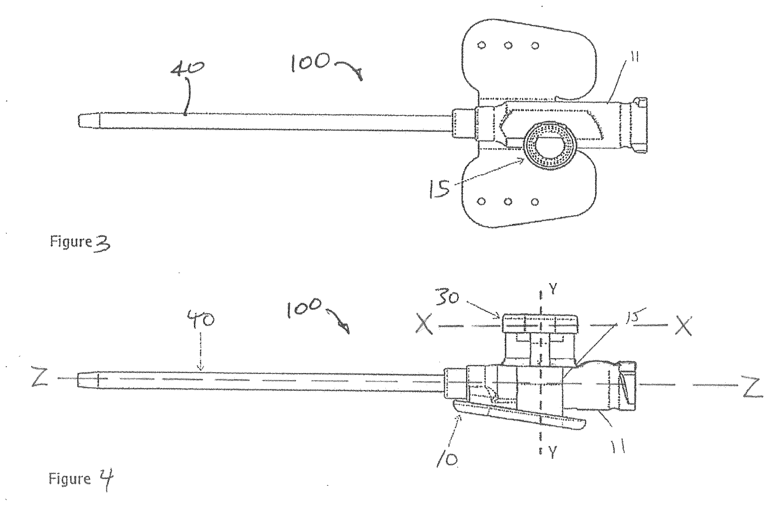

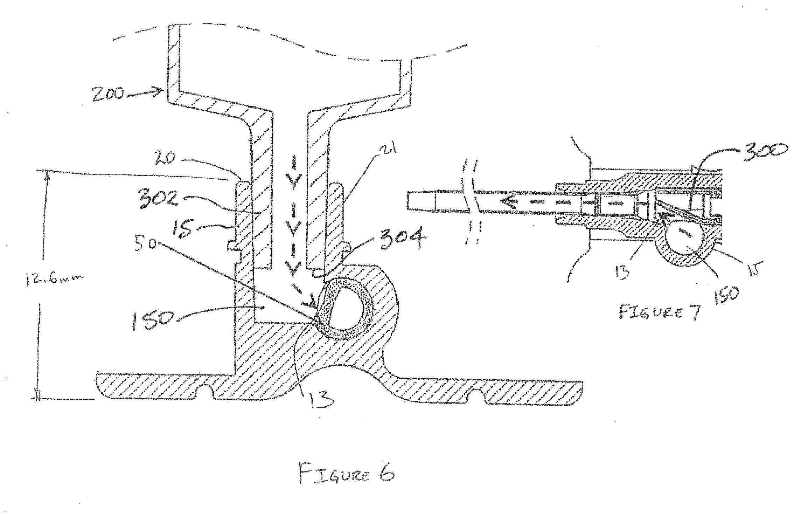

Figures 3 to 5 show the injection port 15 having an axis that is directed generally perpendicular to a lengthwise axis of the catheter body 11 in an offset position. However, the injection port 15 can extend out from the catheter body 11 in any direction other than perpendicular. The overall height of the catheter assembly 100 with the offset port, measured between the lowest point and the highest point, is reduced compared to an in-line injection port arrangement. The height at the top of the injection port 15 is minimized because the central axis of the injection port 15 is displaced from the central axis of the catheter hub 10. Thus, the present assembly has a low profile compared to an in-line injection port arrangement. Another benefit of an offset injection port is in allowing a connection with a male medical implement, such as a syringe 200, to extend deeper into the catheter hub 10, as shown in Figures 6 to 10. The lumen 150 of the injection port 15 communicates with the interior cavity 13 of the catheter body 11 to form an enlarged interior cavity section 300, as shown in Figure 7. That is, the width at the intersection is roughly the cumulative value of the width of the main hub body 11 and the width of the lumen 150 of the injection port 15 at the intersection. If the upper most section of the main body 11 is classified as a 12 o'clock position, then the offset configuration allows a male implement or fluid pressure discharged from the male implement to contact or impact a valve 50 located in the main interior cavity of the hub body 11 at the 8 o'clock to about the 11 o'clock position, as shown in the end cross-sectional view of Figure 6. Thus, the diameter of the lumen 150, the offset distance between the axes of the injection port 15 and the catheter body 11, and the height of the injection port 15 can influence how a male medical implement or fluid pressure discharged therefrom can interact with the valve 50.

With reference again to Figures 2 and 6, a valve 50 is provided to seal the lumen 150 of the injection port 15 from the distal opening and the proximal opening of the main body 11. The valve 50, which is preferably an elastomeric sleeve, has an open bore and permits fluid communication thereacross, which is convention in the art. In the present example, the valve 50 seals off the interface between the lumen 150 and the interior cavity 13 of the hub body 11 by forming a seal to prevent fluid or other contaminants from entering the interior cavity 13 from the injection port 15, and also prevents fluid from passing through the proximal end of the catheter hub 10 from the injection port, and vice versa. For example, if the catheter tube 40 was inserted into the patient, blood from the patient would be sealed off from the injecting port 15, and fluid entering through the proximal end 12 of the catheter hub 10, such as IV solution delivered to the patient, would be prevented from entering the injection port 15. Similarly, any fluid in the injection port 15 cannot pass through the seal formed by the valve 50 to flow into the catheter tube 40 unless the valve is collapsed, as further discussed below. In one example, the valve 50 is a silicone tube.

Figures 6 and 7 are cross-sectional views of one example of a ported catheter assembly 100. The injection port 15 of the catheter hub 10 may have external threads (not shown) at the outer surface 21 surrounding the inlet 20 of the injection port 15. In the illustrated example, the injection port 15 is sized and shaped to accommodate a Luer tip 302 with or without a threaded collar for engaging external threads. The tip 302 of the male medical implement 200 is shown inserted into the inlet 20 of the injection port 15 and due to the relative geometries of the Luer, extends into the lumen 150 a known amount or distance that can easily be sized and computed. As shown, the distal end 304 of the Luer tip 302 is spaced from the valve 50. Fluids stored within the male medical implement 200 can now be injected through the lumen 150 of the injection port 15, the interior cavity 13 of the catheter hub 10, and the catheter tube 40 to the patient. When fluids are discharged from the medical implement 200, fluid pressure presses upon the wall of the valve 50 and collapses the valve 50, breaking the seal, and creating a flow path between the lumen 150 and the distal end 14 of the body and the catheter tube 40. When an IV line is connected to the hub body 11 and fluids are introduced, the discharge pressure from the medical implement 200 must exceed the pressure in the interior cavity 13 of the catheter hub 10 to deflect to the valve 50 to open the valve at the injection port 15. Upon retraction of the male medical implement 200 away from the injection port 15, the elasticity of the valve 50 will cause the valve 50 to move from its collapsed position shown in Figure 8 to its natural cylindrical position to close the fluid pathway between the lumen 150 and the catheter hub body 11.

Referring now to Figure 8, a valve opener 55 is shown disposed inside the lumen 150 of the injection port 15 according to an embodiment of the invention. The valve opener 55 may be sized and shaped to be advanced by the end 304 of the tip 302 of the male medical implement 200 to press against and elastically deform the valve 50. For example, rather than relying on only fluid pressure to collapse the valve 50, as discussed above, the valve opener 55 may be used to physically deform the valve 50 to break the seal and allow fluid to pass from the male medical implement 200 into the patient via the injection port. Advantageously because the valve 50 is physically collapsed by the valve opener 55, a vacuum can be applied or generated at the lumen 150 of the injection port 15 while still maintaining the valve 50 in the collapsed state. Accordingly, in one embodiment, when the valve 50 is physically deformed as shown in Figure 8, fluid can be aspirated through the injection port 15 and into the male medical implement 200. This is not possible for prior art ported catheters as any vacuum pulled at the injection port 15 will cause the valve to restore its shape and block off or seal off the injection port from the main body 11. Thus, an aspect of the present disclosure is understood to include a ported catheter assembly wherein fluids can be injected or aspirated through the injection port 15 of the catheter body 11. In an example, fluids are aspirated through the injection port by first collapsing the valve in the catheter body through physical means or a physical object. As described, a valve opener may be located inside the injection port and advanced into the valve 50 by a medical implement to collapse the valve. Once the valve 50 is collapsed by a physical object rather than fluid pressure, the seal at the injection port is broken and fluids can be injected or aspirated through the injection port 15. Thus, with the present ported catheter, fluid samples may be drawn through, such as aspirated through, the injection port and into a medical implement 200, such as a syringe.

When the physical constraint of the medical implement 200 is removed from the injection port and away from the valve opener 55, the valve 50 elastically returns back to its original shape thereby restoring the seal between the lumen 150 of the injection port 15 and the interior cavity 13 of the catheter body 11. Thus, the lumen 150 of the injection port 15 is no longer in fluid communication with the interior cavity 13 of the catheter body 11 due to the seal formed by the valve 50. Said differently, aspects of the present disclosure include a valve that elastically springs back to its original cylindrical shape to move a valve opener located inside an injection port of a ported catheter back to its ready position, so that the valve can be opened and closed multiple times. The injection port having the valve opener moved by the valve can be offset from the lengthwise axis of the hub body or be in-line with the lengthwise axis, as further discussed below.

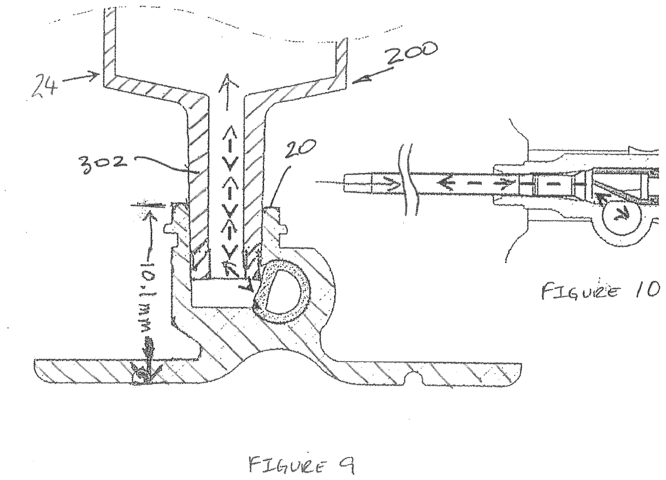

Figures 9 and 10 illustrate another embodiment of the catheter assembly 10 which is similar to the example of Figures 6 and 7 except that the injection port 15 of the present embodiment includes a modified port that has been shortened even further. In the embodiment shown, the injection port does not show external threads. Instead of a threaded female Luer at the injection port, the ISO taper standard is retained with the port 15 but the port is reduced in length to allow the male Luer tip 302 of the medical implement 200 to be inserted deeper into the injection port without the threaded collar blocking or interfering with the insertion. Thus, the inlet 20 is configured for a Luer slip syringe or connector, but only a modified Luer lock syringe or connector with shortened threads. Using the modified port geometry and the offset port arrangement, the tip 302 of the male medical implement 200 is able to contact and presses against the valve 50 to physically collapse the valve 50 without a valve opener. Thus, unlike the embodiment of Figure 8, the present embodiment is configured to permit the tip 302 of the medical implement to directly abut or push on the valve 50 to collapse the valve and break the seal formed between the lumen 150 of the injection port and the interior cavity 13. Thus the physical contact between the male medical implement 200 and the valve 50 opens a pathway between the lumen 150 of the injection port 15 and the interior cavity 13 of the catheter body 11 for fluid communication between the injection port 15 and the interior cavity 13 of the catheter body 11. Therefore, when a vacuum is provided or generated at the inlet or in the lumen of the injection port 15, the valve will not reseal the lumen 150 from the interior cavity of the catheter body 11. Fluid can now be injected into the patient through the injection port and/or blood can be withdrawn or aspirated from the patient through the injection port 15. When the tip of the medical male implement 200 is no longer pressed against the valve 50, such as by removing the tip 302 from the lumen, the valve 50 will elastically return back into its original shape thereby restoring the seal between the lumen 150 of the injection port 15 and the interior cavity 13 of the catheter body 11. Thus, the lumen 150 of the injection port 15 is no longer in fluid communication with the interior cavity 13 of the catheter body 11 once the valve returns to its original shape from its collapsed position.

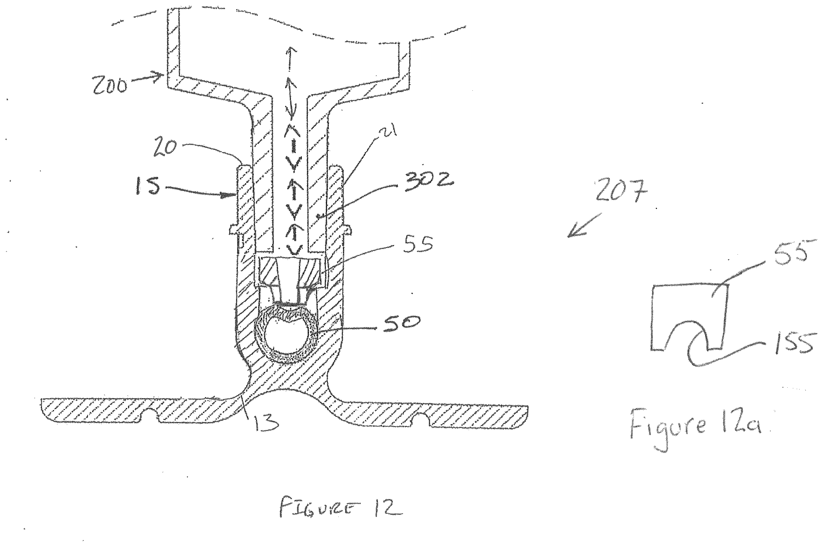

Referring now to Figure 11, another example of a ported catheter assembly 207 is shown, which is similar to the catheter assembly 100 with an offset injection port 15 except that the axis of the injection port 15 of the present example intersects the axis of the catheter body 10. The ported catheter assembly 207 of the present example includes a catheter hub 10 having a catheter body 11 and an injection port 15 extending from the catheter body 11. A port cap assembly 30 is provided to cap the injection port 15 and a valve 50 is provided for the interior of the catheter hub 10 to isolate the lumen 150 from the interior cavity 13 of the hub body 11. The assembly may comprise a valve opener in an analogous manner to that that shown in Figure 8 and discussed above or as shown in Figure 12 and discussed below. A catheter tube 40 is also shown and is to be attached to the catheter hub 10 via the fitting 45. The ported catheter assembly 207 is shown without a needle hub and a needle for inserting the catheter tube 40 into a patient. Thus, an aspect of the present disclosure is understood to include a ported catheter hub with a catheter tube, a needle projecting through the catheter tube with the needle tip extending distally of the distal opening of the catheter tube, and a needle hub attached to the needle and located proximally of the catheter hub. In some examples, a third hub having a tip guard for covering the tip following successful venipuncture is provided between the catheter hub and the needle hub. In still other examples, the needle guard is provided with the needle hub so that following successful venipuncture, the needle and needle tip is retracted into the needle hub, such as by spring action. In still other examples, a spring clip is engaged inside the catheter hub until the needle tip is retracted inside the spring clip, which then collapses and blocks the needle tip, while disengaging from the inside of the catheter hub. In this last example, the needle can have a change in contour proximal of the needle tip which is blocked from passing through an opening in a proximal wall of the spring clip.

Referring now to Figure 12, the injection port 15 is configured to accommodate a tip in a Luer lock or a Luer slip arrangement. The injection port 15 of the catheter hub 10 can have external threads (not shown) at the outer surface 21 surrounding the inlet 20 of the injection port 15. The tip 302 of the male medical implement 200 is shown inserted into the inlet 20 of the injection port and fluids discharged from the male medical implement can flow through the port 15 and into the patient through the lumen 150 of the injection port 15, the interior cavity 13 of the catheter hub 10, and the catheter tube 40.

As shown, a valve opener 55 is provided inside the lumen 150 of the injection port 15. The tip 302 pushes the valve opener 55 into the valve 50 to physically deform the valve 50 to then break the seal and allow fluid to pass to or from the patient through the injection port 15. The injection port 15 remains opened due to the valve opener 55 collapsing the valve 50, even if a vacuum is pulled by the medical implement 200 at the injection port 15.

When the medical implement 200 is removed from the injection port and the valve opener 55 no longer held by the constraint of the tip 302, the elasticity of the valve 50 causes it to return to its cylindrical shape and push the valve opener up to its ready position, which then seals the lumen 150 of the injection port 15 to isolate the lumen from the interior cavity 13 of the catheter body 11. As the end of the valve opener 55 can press into and seals against the valve 50, in some examples, the end of the valve opener 55 that contacts the valve 50 may be provided with one or more projections to form flow channels for fluid flow from or to the medical implement. This arrangement of the valve opener 55 is shown in Figure 12a, which shows a side view of the valve opener 55. Arched openings 155 are seen in the middle of the bottom surface of the cylindrical valve opener 55. These openings avoid that the valve opener seals against the valve 50 and allows for a free flow in both directions. In other examples, instead of or in addition to the one or more projections, the tip of the valve opener that contacts the valve or the tip of the medical implement or both may incorporate slots or channels for fluid flow, as further discussed below.

In another example, the injection port 15 is a modified port, similar to that discussed with reference to FIG. 9. The modified port is shortened so that the injection port 15 is configured for a Luer fit only, not a Luer lock. This allows a tip 302 of a medical implement to physically contact the valve 50 directly without a valve opener to collapse the valve. The tip of the medical implement should include one or more projections and/or one or more slots for fluid flow. Thus, an in-line injection port in which the axis of the injection port is in-line with a lengthwise axis of the catheter body, such as a centered injection port, can still be used to both inject and aspirate fluid without a valve opener when using a modified Luer slip port with a shorter than standard port length.

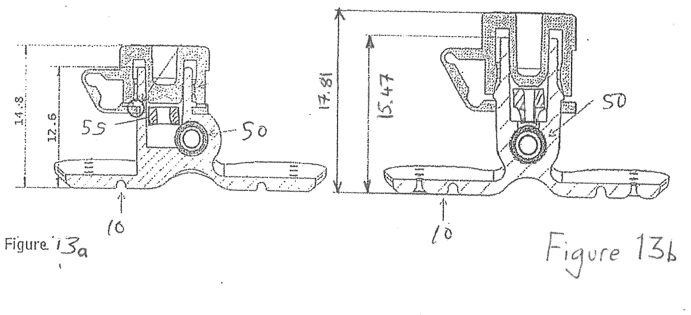

Referring now to FIG. 13, a ported catheter with an offset injection port and a ported catheter with a centered injection port are shown side-by-side. The overall height of the ported catheter assembly 100 with the offset injection port 15 is less than a ported catheter assembly 100 with a centered injection port. In the illustrated embodiment, the height of the ported catheter assembly 100 with the offset injection port is about 14.8 mm from the port cap assembly 30 to the bottom of the wings 19, and the height of the ported catheter assembly 100 is about 12.6 mm from the offset injecting port 15 to the bottom of the wings 19. The depth of the injection port 15 can remain relatively the same but because it is offset from center, the height of the injection port can be smaller than known ports, resulting in a smaller package. The height of the centered injection port is about 17.8 mm from the port cap assembly 30 to the bottom of the wings 19. Thus the offset port with closed cap is 3 mm (17%) shorter than known ports. This creates a more stable and secure IV access port on the patient, which minimizes the risk of it being knocked or caught and ripped out of the vein.

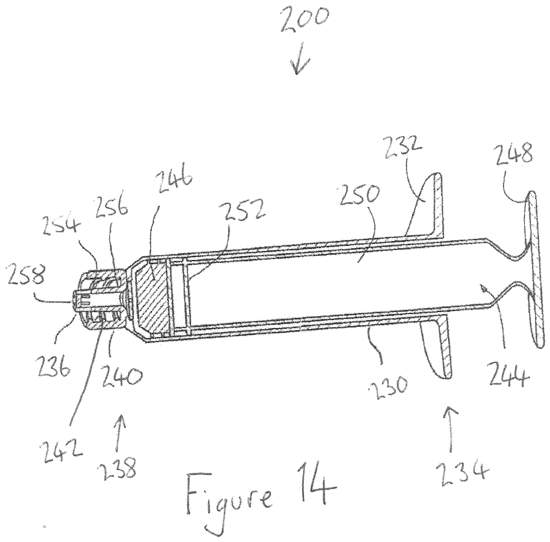

Referring now to FIG. 14, there is shown a syringe 200. The syringe 200 comprises a barrel 230 having a gripping flange 232 at a proximal end 234 and a syringe tip 236 at a distal end 238, which is preferably a Luer tip for mating engagement with a female Luer, such as a catheter hub, a needleless port access valve, a male Luer connector, and the like. The syringe 200 includes a collar 240 with internal threads 242 for use as a Luer lock. However, the syringe 200 may be practiced without the threaded collar, for use as a Luer slip. A piston or plunger 244 has a plunger tip 246 and a push flange 248 is slidably positioned inside the syringe barrel 230 for aspirating or expelling fluids from the barrel. In one example, the plunger 244 is made from a plurality of longitudinal ribs 250 and radial ribs 252. In another example, the plunger is made from a central core or rod with one or more ribs and without a plunger tip. In other words, the plunger stem and plunger tip are integrally formed and is commonly referred to as a two-piece syringe in the industry. The syringe of Figure 14 is commonly referred to as a three-piece syringe in the industry.

The syringe 200 may be made from a number of known prior art plastic materials using known methods. The syringe 200 is preferably made from a transparent or semitransparent material to permit viewing through the syringe barrel. Less preferred, the syringe barrel is made from an opaque material. Graduated marks, scale, logo, and/or other indicia, such as lot number, may be printed on the outside of the syringe barrel for reference purposes.

With reference to Figure 14, the syringe tip 236 comprises an elongated body 254 comprising a base 256 connected to the distal end 238 of the syringe barrel 230 and a tip end 258 having a generally planar end surface, which may be practiced with an arc or taper. In the example shown, a central section of the tip end 258 is solid and does not incorporate a lumen. Instead, multiple flow channels are incorporated radially of the central portion. In one example, the flow channels are each generally rectangular or rectilinear in shape and includes at least a cut-out formed on or through the tip end 258. More preferably, each flow channel is formed by a cut-out formed through both the tip end 258 and the tip body 254, at the intersection thereof. Although six flow channels are shown in Figure 14, fewer or more than six flow channels may be incorporated.

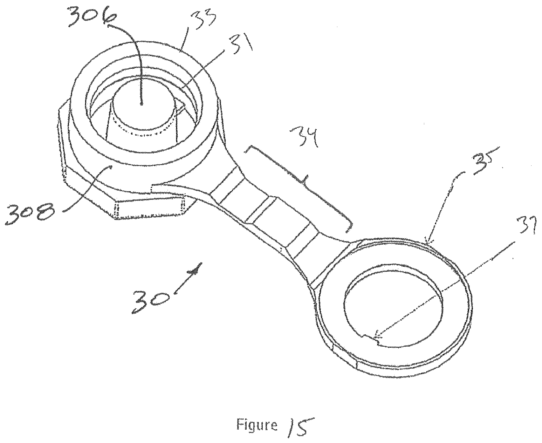

Referring now to Figure 15, the port cap assembly 30 is shown for use to close an end of an injection port 15. The port cap assembly 30 includes a sealing top 33 for closing the port 15, a securing ring 35 to secure the sealing top 33 to the port, and a hinge 34 connecting the sealing top 33 to the securing ring 35 so that that sealing top 33 can be held to the catheter hub 10 when the port 15 is opened, which helps to prevent loss or misplacement of the sealing top 33. The sealing top 33, the hinge 34, and the securing ring 35 can be integrally formed and the hinge 34 can be a living hinge. The hinge 34 can also act as a spring when elastically bent. Thus, the sealing top 33 could swing away from the injection port 15 caused by the restoring elastic force of the hinge. The port cap assembly 30 can be made of any biocompatible material, such as plastic.

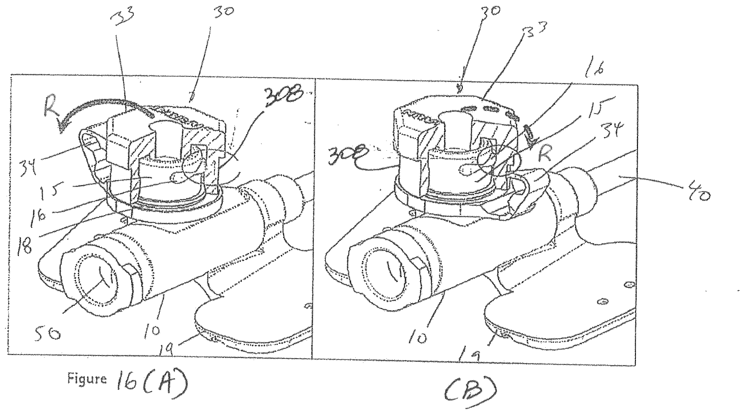

The sealing top 33 has a male tip 306 and a protruding ring 31 inside a skirt section 308. The protruding ring 31 is configured to engage with the notch 16 (Figures 1 and 16) on the port 15 to generate resistance when removing the sealing top 33 from the injection port 15 from the closed position. For example, the opening movement is designated by the arrows R in Figure 16(A) and Figure 16(B). In the closed position of Figure 16(B), the top 33 is rotated so that the hinge 34 aligns with the notch 16. Internally of the skirt 308, the ring 31 (see Figure 15) engages the notch 16 to prevent separation of the sealing top 33 from the port. To open the top 33, the top is pulled in the direction of the arrow R, as shown in Figure 16(A) or 16(B). Internally of the skirt 308, the ring 31 moves away from the notch 16 so that the top 33 can now open. The protruding ring 31 can have a chamfer to allow the protruding ring to glide on the notch to ease rotation into the closed position from the open position. A user closing the port cap assembly 30 may need to press the sealing top 33 slightly towards the injection port 15 to engage the protruding ring 31 into or against the notch 16.

The securing ring 35 can be secured to the injection port 15 by stretching elastically past the flange 18 of the injection port 15 and snapping the ring into place. The outer diameter of the flange 18 is larger than an inner diameter of the securing ring 35 to secure the securing ring 35 behind the flange after inserting it into place. The flange 18 holds the securing ring 35 in place but the securing ring 35 can rotate freely about the axis of the injection port. The securing ring 35 can be made of polyethylene or polypropylene material. The port cap assembly 30 can be made entirely from polyethylene or polypropylene.

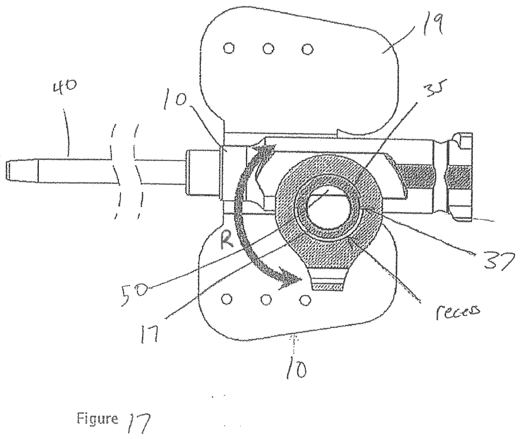

Figure 17 shows a protruding tab 37 extending inwardly from the securing ring 35 and engages with the track 17 by gliding along the track 17 to limit rotation of the port cap assembly 30, such as from 0 to 180 degrees. The rotational range is not limited to 0 to 180 degrees. For example, the cap can be rotated from 0 to less than 180 degrees such as 90 degrees or more than 180 degrees, such has 270 degrees. The preferred degree of rotation is as shown, in which the hinge can be rotated from one side to the other around the distal side of the port 15. It is preferred not to rotate the hinge to the proximal side of port 15. If the cap were to be pulled open when the hinge was on the proximal side of the port, then the pulling action would be in a direction to pull the catheter out of the vein. The protruding tab 37 acts like a pinion on a rack where it is sitting in the track 17. In another example, the track 17 can be on the port cap assembly 30 and the protruding tab 37 on the catheter body 11. Other means to limit rotation of the port cap assembly 30 are contemplated.

In a method of using the ported catheter assembly 100, the ported catheter assembly 100 is assembled with a needle hub having a needle passing through the interior cavity 13, the valve 50, the bushing 45, the catheter 40, and into a patient's vein. The needle hub is then removed after the catheter 40 is inserted into the patient's vein. The needle hub is replaced with a connector. The connector can allow a solution, such as an IV solution, to be infused into the patient.

When a bolus injection is to be delivered to the patient, the port cap assembly 30 is unsnapped from the port 15. The sealing top 33 can then be placed to the side of port 15 thereby exposing the lumen 150 of the injection port 15. A medical injection instrument, such as a syringe 200, can be inserted into the injection port 15 to deliver the medicament through the injection port 15, the catheter hub 10, and the catheter tube to the patient. The tip of the syringe or a valve opener 55 located in the injection port can press against the valve 50 thereby breaking a seal between the lumen 150 of the injection port 15 and the interior cavity 13 of the catheter hub 10 by deforming the valve 50 to create a flow path for fluid. Alternatively, pressure generated by the medical injection instrument may be sufficient to press open the valve 50 to create the fluid flow path without physically collapsing the valve, such as collapsing only by fluid pressure. The medicament is then delivered from the chamber or barrel of the medical implement through the tip 302 and out the side opening 220 and/or the distal central opening of the tip into the interior cavity 13 of the catheter hub 10. If desired, fluid, such as blood, can be drawn from the patient through the injection port as the valve 50 is physically pressed open and not opened only by fluid pressure. If the valve 50 is pressed open by fluid pressure, then only the contents of the medical implement 200 can be delivered to the patient.

When delivery is complete, the syringe 200 can be removed and the valve restores elastically back to seal off the injection port 15 from the interior cavity 13 of the catheter hub 10. The sealing top 33 is then snapped back on the injection port to cover it and prevent contaminants from entering the injection port 15.

Referring to Figure 18, there is shown the ported catheter assembly of Figure 4 with a spring clip needle guard 500 located within the catheter hub 10. The needle guard comprises a proximal wall 502. Resilient arms 504 and 506 extend in the distal direction from the proximal wall and terminate in respective distal end walls 508 and 510. The distal end portion of each arm 504, 506 engages in a groove 520 formed in the inner surface of the catheter hub 10. The proximal wall 502 comprises an opening therein for receiving a needle 530. The needle 530 is shown in Figure 18 in the ready position, in which the shaft of the needle extends through the opening in the proximal wall 502 of the needle guard 500, through the catheter hub and through the catheter 40, such that the needle tip extends distally of the distal end of the catheter 40. The needle 530 is provided with a bulge 532 in its shaft. In the ready position, the bulge 532 is located within the catheter 40, as shown. As can be seen, in the ready position, the distal end walls 508, 510 of the needle guard 500 engage with the shaft of the needle 530, the action of which keeps the distal end portions of the arms 504, 506 engaged with the groove 520 in the inner surface of the catheter hub 10.

When the needle 530 is retracted in the distal direction (that is to the right in Figure 18), the needle tip passes the distal guard walls 508, 510 of the needle guard, allowing the arms 504, 506 to spring inwards to block the needle tip, releasing the engagement with the groove 520 in the catheter hub. The needle and the needle guard may then be removed together from the catheter hub 10. The bulge 532 in the needle shaft engages with the opening in the proximal wall 502 of the needle guard 500, to prevent the needle guard 500 leaving the needle shaft. The needle and needle guard are shown in the protected position removed from the catheter hub in Figure 19.

Other types of spring clip needle guards can be used. For example the spring clip needle guard can have two arms, with only one arm provided with a distal guard wall 508 or 510, which blocks the needle tip, when the needle is retracted into the spring clip. Another example is a spring clip with only one arm 504 or 506. Alternatively, the spring clip may comprise an arm that extends between the needle and the catheter hub inner wall.

The bulge 532 can be a spherical bulge or a bulge created by crimping, wherein the round needle shaft is squeezed in at two diametrically opposed positions and bulges out in the two perpendicular diametrically opposed positions.

Methods of making ported catheter assemblies as described herein are contemplated.

## Claims

1. A ported catheter assembly (100, 207), comprising:
a catheter hub (10) having a distal end, an interior cavity, and a proximal end;
a port (15) extending from the catheter hub (10) and having an inner opening communicating with the interior cavity of the catheter hub (10);
a catheter (40) extending from the distal end of the catheter hub (10);
a needle projecting through the catheter tube (40) with the needle tip extending distally of the distal opening of the catheter tube and
a valve (50) providing a seal between the interior cavity of the catheter hub (10) and the port (15) when the valve (50) is in a closed position;
wherein the valve (50) has an open position, in which two-way fluid flow is permitted between the interior cavity of the catheter hub (10) and the port (15), the valve (50) being moved to the open position by the insertion of an instrument into the port (50) wherein
a valve opener (55) is disposed in the port (15) or the port (15) is configured in that the instrument, when inserted, directly contacts the valve (50),
wherein the valve opener (55) is urged by an instrument inserted into the port (15) into contact with the valve (50) to move the valve (50) from the closed position to the open position, wherein the valve opener (55) comprises a valve opener body, which comprises a bore therethrough for the passage of fluid between the instrument and the interior cavity of the catheter hub (10).

2. The ported catheter assembly (100, 207) according to claim 1, wherein a portion of the valve (50) is moveable inwardly from the closed position to the open position.

3. The ported catheter assembly (100, 207) according to any one of the preceding claims, wherein the valve opener body is generally cylindrical.

4. The ported catheter assembly (100, 207) according to any one of the preceding claims, wherein the valve opener body has an actuating end for contacting the valve (50).

5. The ported catheter assembly (100, 207) according to claim 4, wherein the actuating end is provided with one or more openings extending therefrom along the valve opener body.

6. The ported catheter assembly (100, 207) according to claim 5, wherein the or each opening is arched.

7. The ported catheter assembly (100, 207) according to any of claims 4 to 6, wherein the actuating end of the valve opener body is tapered.

8. The ported catheter assembly (100, 207) according to claim 7, wherein the diameter of the inner opening of the port (15) is less than the diameter of the lumen of the port (15), thereby defining a shoulder within the port (15), the diameter of the valve body being greater than the diameter of the inner opening, the tapered actuating end of the valve opener body allowing the actuating end to pass through the inner opening and contact the valve (50).

9. The ported catheter assembly (100, 207) according to any one of the preceding claims, wherein the valve (50) is biased into the closed position.

10. The ported catheter assembly (100, 207) according to claim 9, wherein the valve (50) is resilient, the resilience of the valve biasing the valve (50) into the closed position.

11. The ported catheter assembly (100, 207) according to any one of the preceding claims, wherein the valve (50) is made of silicone.

12. The ported catheter assembly (100, 207) according to any one of the preceding claims, wherein the valve (50) is disposed within the interior cavity of the catheter hub (10).

13. The ported catheter assembly (100, 207) according to claim 12, wherein the valve (50) comprises a generally cylindrical valve body, a portion of the valve body extending across the inner opening of the port (15).

14. The ported catheter assembly (100, 207) according to claim 13, wherein the outer surface of the valve body is in contact with the inner surface of the interior cavity of the catheter hub (10) to form a fluid-tight seal.

15. The ported catheter assembly (100, 207) according to any one of the preceding claims, wherein a pair of wings (19) extends from the catheter hub (10).

## Patentansprüche

1. Katheteranordnung mit Port (100, 207) umfassend:
einen Katheteransatz (10), der ein distales Ende, einen inneren Hohlraum und ein proximales Ende aufweist;
ein Port (15), der sich von dem Katheteransatz (10) erstreckt und eine innere Öffnung aufweist, die mit dem inneren Hohlraum des Katheteransatzes (10) in Verbindung steht;
einen Katheter (40), der sich von dem distalen Ende des Katheteransatzes (10) erstreckt;
eine Nadel, die sich durch den Katheterschlauch (40) erstreckt, wobei die Nadelspitze distal von der distalen Öffnung des Katheterschlauchs ragt und
ein Ventil (50), das eine Abdichtung zwischen dem inneren Hohlraum des Katheteransatzes (10) und der Öffnung (15) bereitstellt, wenn sich das Ventil (50) in einer geschlossenen Position befindet;
wobei das Ventil (50) eine offene Position aufweist, in der ein Zwei-Wege-Flüssigkeitsdurchfluss zwischen dem inneren Hohlraum des Katheteransatzes (10) und dem Port (15) ermöglicht wird, wobei das Ventil (50) durch das Einführen eines Instruments in den Port (50) in die offene Position bewegt wird,
wobei ein Ventilöffner (55) in dem Port (15) derart angeordnet oder der Port (15) derart konfiguriert ist, dass das Instrument, wenn es eingeführt wird, das Ventil (50) direkt berührt,
wobei der Ventilöffner (55) durch ein in den Port (15) eingeführtes Instrument in Berührung mit dem Ventil (50) gedrückt wird, um das Ventil (50) von der geschlossenen Position in die offene Position zu bewegen, wobei der Ventilöffner (55) einen Ventilöffnerkörper mit einer Durchbohrung für den Durchfluss von Flüssigkeit zwischen dem Instrument und dem inneren Hohlraum des Katheteransatzes (10) aufweist.

2. Katheteranordnung mit Port (100, 207) nach Anspruch 1, wobei ein Abschnitt des Ventils (50) von der geschlossenen Position in die offene Position nach innen beweglich ist.

3. Katheteranordnung mit Port (100, 207) nach einem der vorhergehenden Ansprüche, wobei der Ventilöffnerkörper im Allgemeinen zylindrisch ist.

4. Katheteranordnung mit Port (100, 207) nach einem der vorhergehenden Ansprüche, wobei der Ventilöffnerkörper ein Betätigungsende zum Berühren des Ventils (50) aufweist.

5. Katheteranordnung mit Port (100, 207) nach Anspruch 4, wobei das Betätigungsende mit einer oder mehreren Öffnungen versehen ist, die sich davon entlang des Ventilöffnerkörpers erstrecken.

6. Katheteranordnung mit Port (100, 207) nach Anspruch 5, wobei die oder jede Öffnung gewölbt ist.

7. Katheteranordnung mit Port (100, 207) nach einem der Ansprüche 4 bis 6, wobei das Betätigungsende des Ventilöffnerkörpers verjüngt ist.

8. Katheteranordnung mit Port (100, 207) nach Anspruch 7, wobei der Durchmesser der inneren Öffnung des Ports (15) kleiner als der Durchmesser des Lumens des Ports (15) ist, wodurch eine Schulter innerhalb des Ports (15) definiert wird, wobei der Durchmesser des Ventilkörpers größer als der Durchmesser der inneren Öffnung ist, wobei das verjüngte Betätigungsende des Ventilöffnerkörpers ermöglicht, dass das Betätigungsende durch die innere Öffnung passt und das Ventil (50) berührt.

9. Katheteranordnung mit Port (100, 207) nach einem der vorhergehenden Ansprüche, wobei das Ventil (50) in die geschlossene Position vorgespannt ist.

10. Katheteranordnung mit Port (100, 207) nach Anspruch 9, wobei das Ventil (50) elastisch ist, und wobei die Elastizität des Ventils das Ventil (50) in die geschlossene Position vorspannt.

11. Katheteranordnung mit Port (100, 207) nach einem der vorhergehenden Ansprüche, wobei das Ventil (50) aus Silikon hergestellt ist.

12. Katheteranordnung mit Port (100, 207) nach einem der vorhergehenden Ansprüche, wobei das Ventil (50) innerhalb des inneren Hohlraums des Katheteransatzes (10) angeordnet ist.

13. Katheteranordnung mit Port (100, 207) nach Anspruch 12, wobei das Ventil (50) einen im Allgemeinen zylindrischen Ventilkörper umfasst, wobei sich ein Abschnitt des Ventilkörpers über die innere Öffnung des Ports (15) hinweg erstreckt.

14. Katheteranordnung mit Port (100, 207) nach Anspruch 13, wobei die Außenoberfläche des Ventilkörpers mit der Innenoberfläche des inneren Hohlraums des Katheteransatzes (10) in Berührung steht, um eine fluiddichte Abdichtung auszubilden.

15. Katheteranordnung mit Port (100, 207) nach einem der vorhergehenden Ansprüche, wobei sich ein Paar Flügel (19) von dem Katheteransatz (10) erstreckt.

## Revendications

1. Ensemble cathéter à orifice (100, 207), comprenant :
une embase de cathéter (10) ayant une extrémité distale, une cavité intérieure et une extrémité proximale ;
un orifice (15) s'étendant depuis l'embase du cathéter (10) et communiquant avec la cavité intérieure de l'embase de cathéter (10) ;
un cathéter (40) s'étendant depuis l'extrémité distale de l'embase du cathéter (10) ;
une aiguille faisant saillie à travers le tube de cathéter (40), la pointe d'aiguille s'étendant de manière distale par rapport à l'ouverture distale du tube de cathéter et
une valve (50) fournissant un joint entre la cavité intérieure de l'embase de cathéter (10) et l'orifice (15) lorsque la valve (50) est dans une position fermée ;
la valve (50) ayant une position ouverte, un écoulement de fluide bidirectionnel étant autorisé entre la cavité intérieure de l'embase de cathéter (10) et l'orifice (15), la valve (50) étant déplacée vers la position ouverte par l'insertion d'un instrument dans l'orifice (50)
un dispositif d'ouvre-valve (55) étant disposé dans l'orifice (15) ou l'orifice (15) étant conçu en ce que l'instrument, lorsqu'il est inséré, entre directement en contact avec la valve (50),
le dispositif d'ouvre-valve (55) étant poussé par un instrument inséré dans l'orifice (15) en contact avec la valve (50) pour déplacer la valve (50) de la position fermée à la position ouverte, le dispositif d'ouvre-valve (55) comprenant un corps de dispositif d'ouvre-valve, qui comprend un alésage à travers celui-ci pour le passage de fluide entre l'instrument et la cavité intérieure de l'embase de cathéter (10).

2. Ensemble cathéter à orifice (100, 207) selon la revendication 1, une partie de la valve (50) étant mobile vers l'intérieur de la position fermée à la position ouverte.

3. Ensemble cathéter à orifice (100, 207) selon l'une quelconque des revendications précédentes, le corps de dispositif d'ouvre-valve étant généralement cylindrique.

4. Ensemble de cathéter à orifice (100, 207) selon l'une quelconque des revendications précédentes, le corps de dispositif d'ouvre-valve ayant une extrémité d'actionnement pour entrer en contact avec la valve (50).

5. Ensemble cathéter à orifice (100, 207) selon la revendication 4, l'extrémité d'actionnement étant pourvue d'une ou de plusieurs ouvertures s'étendant à partir de celle-ci le long du corps de dispositif d'ouvre-valve.

6. Ensemble cathéter à orifice (100, 207) selon la revendication 5, l'une ou chaque ouverture étant arquée.

7. Ensemble cathéter à orifice (100, 207) selon l'une quelconque des revendications 4 à 6, l'extrémité d'actionnement du corps de dispositif d'ouvre-valve étant effilée.

8. Ensemble cathéter à orifice (100, 207) selon la revendication 7, le diamètre de l'ouverture intérieure de l'orifice (15) étant inférieur au diamètre de la lumière de l'orifice (15), définissant ainsi un épaulement à l'intérieur de l'orifice (15), le diamètre du corps de valve étant supérieur au diamètre de l'ouverture intérieure, l'extrémité d'actionnement conique du corps de dispositif d'ouvre-valve permettant à l'extrémité d'actionnement de passer à travers l'ouverture intérieure et d'entrer en contact avec la valve (50).

9. Ensemble cathéter à orifice (100, 207) selon l'une quelconque des revendications précédentes, la valve (50) étant sollicitée dans la position fermée.

10. Ensemble cathéter à orifice (100, 207) selon la revendication 9, la valve (50) étant élastique, l'élasticité de la valve sollicitant la valve (50) en position fermée.

11. Ensemble cathéter à orifice (100, 207) selon l'une quelconque des revendications précédentes, la valve (50) étant en silicone.

12. Ensemble cathéter à orifice (100, 207) selon l'une quelconque des revendications précédentes, la valve (50) étant disposée à l'intérieur de la cavité intérieure de l'embase de cathéter (10).

13. Ensemble cathéter à orifice (100, 207) selon la revendication 12, la valve (50) comprenant un corps de valve généralement cylindrique, une partie du corps de valve s'étendant à travers l'ouverture intérieure de l'orifice (15).

14. Ensemble cathéter à orifice (100, 207) selon la revendication 13, la surface extérieure du corps de valve étant en contact avec la surface intérieure de la cavité intérieure de l'embase de cathéter (10) pour former un joint étanche aux fluides.

15. Ensemble cathéter à orifice (100, 207) selon l'une quelconque des revendications précédentes, une paire d'ailettes (19) s'étendant depuis l'embase de cathéter (10).
